# EUROPÄISCHE PATENTSCHRIFT

(11) **EP 0 337 435 B1**
(45) Veröffentlichungstag und Bekanntmachung des Hinweises auf die Patenterteilung: **01.02.1995**
(21) Anmeldenummer: 89106531.0
(22) Anmeldetag: 12.04.1989
(51) Int. Cl.: C09B 57/00, C09B 67/10

(54) **Verfahren zur Herstellung deckender Diketopyrrolopyrrolpigmente**
Process for the manufacture of covering diketopyrrolopyrrole pigments
Procédé de fabrication de pigments de dicétopyrrolopyrrole couvrants

(30) Priorität: 15.04.1988 US 181864
(43) Veröffentlichungstag der Anmeldung: 18.10.1989
(73) Patentinhaber: CIBA-GEIGY AG, 4002 Basel (CH)
(72) Erfinder: Jaffe, Edward Ephraim, Dr., Wilmington Delaware 19810 (US); Bäbler, Fridolin, Dr., CH-1723 Marly (CH)
(74) Vertreter: Zumstein, Fritz, Dr.

(56) Entgegenhaltungen:
- EP-A- 101 666
- EP-A- 0 133 156
- EP-A- 0 181 290
- EP-A- 0 221 853
- EP-A- 0 267 877
- EP-A- 0 305 328

## Beschreibung

Die vorliegende Erfindung betrifft ein Verfahren zur Herstellung deckender Diketopyrrolopyrrolpigmente hoher Farbstärke, Farbtonreinheit und Sättigung durch Nassmahlung in einem Alkohol/Base-System.

Aus EP-A 94 911 ist bekannt, dass eine deckendere Pigmentform erhalten werden kann, wenn man Diketopyrrolopyrrolpigmente in Wasser oder einem organischen Lösungsmittel heizt. Organische Lösungsmittel mit Siedepunkt über 80°C, wie z.B. Xylole, Chlorbenzol, Nitrobenzol, Pyridin, Cyclohexanon, Ethylenglykolmonomethylether oder Dimethylformamid sind bevorzugt. In EP-A 190 999 ist ein Verfahren zur Herstellung farbstarker Pigmente, wie z.B. Diketopyrrolopyrrolpigmente, durch Protolyse des durch Behandlung des Rohpigments mit einer starken Base oder bei der Synthese anfallenden Pigmentsalzes beschrieben, welches dadurch gekennzeichnet ist, dass man das Pigmentsalz in einem organischen Lösungsmittel so mit Wasser behandelt, dass das organische Lösungsmittel gleichzeitig während der Protolyse entfernt wird. Die so erhaltenen Pigmente, die eine feinkristalline Form aufweisen, zeichnen sich durch ihre Farbstärke und Transparenz aus. In EP-A 181 290 wird ausgeführt, dass durch Nachbehandlung in Wasser oder in einem organischen Lösungsmittel (vorzugsweise über 80°C) und nachträgliche Zerkleinerung, wie z.B. Nassmahlung, von Diketopyrrolopyrrolpigmenten besonders transparente Pigmentformen erhalten werden. Aus EP-A 101 666 ist ein Verfahren zur Herstellung von organischen Pigmenten mit verbesserter Dispergierbarkeit durch Mahlen in Wasser und 1,2-Diolen mit mindestens 6 C-Atomen, bei bevorzugten Temperaturen zwischen 15 und 70°C bekannt.

Es ist nun gefunden worden, dass durch Nassmahlung bestimmter Diketopyrrolopyrrolpigmente in einem ausgewählten Alkohol/Base-System, ohne zu heizen, ganz überraschend eine deckende Pigmentform erhalten wird, welche sich durch ausserordentliche Farbstärke, hohe Reinheit des Farbtones mit unerwartet hoher Sättigung auszeichnet.

Die vorliegende Erfindung betrifft demnach ein Verfahren zur Herstellung von deckenden 1,4-Diketopyrrolo-[3,4-c]-pyrrole der Formel 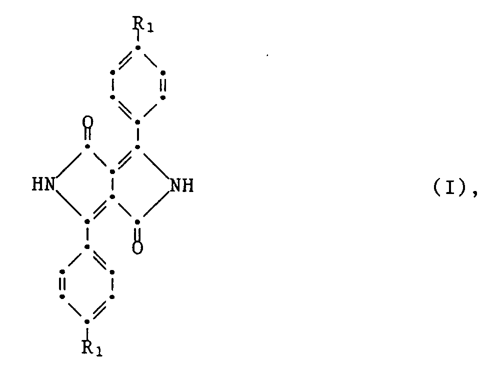
worin R₁ Wasserstoff, Chlor, Methyl oder tert.-Butyl ist, dadurch gekennzeichnet, dass das Pigment in einem Alkohol aus der Gruppe Methanol, Ethanol, Butanol, Pentanol, Ethylenglykol und Propylenglykol in Gegenwart einer Base aus der Gruppe der Alkalimetallhydroxide und quaternären Ammoniumhydroxide, bei einer Temperatur unter 50°C, bevorzugt zwischen 10 und 40°C, gemahlen wird.

Bevorzugt werden Methanol und Ethanol. Geeignete Basen sind z.B. Natriumhydroxid, Kaliumhydroxid, Lithiumhydroxid oder Benzyltrimethylammoniumhydroxid. Bevorzugt werden Natrium- und Kaliumhydroxid.

Der Alkohol ist zweckmässig in einer 5- bis 25-fachen, bevorzugt 10- bis 16-fachen Menge, bezogen auf das Gewicht des Pigments, vorhanden. Ein zweckmässiger Konzentrationsbereich der Base ist von 2 bis 50 %, bevorzugt von 20 bis 40 %, bezogen auf das Gewicht des Pigments. Anorganische Basen können als 15 bis 70%ige, bevorzugt 25 bis 50%ige wässrige Lösungen, organische Basen als 100%ige oder wenigstens als 50%ige wässrige Lösungen eingesetzt werden. Die bevorzugte Konzentration der Base variiert allerdings je nach eingesetztem Alkohol.

Als Apparatur für die Mahlung kann jede Vorrichtung verwendet werden, die es erlaubt, in einem flüssigen Medium das Pigment und den Trägerstoff intensiven mechanischen Kraftwirkungen zu unterwerfen. Derartige Apparaturen sind in grösserer Zahl bekannt. Sie beruhen beispielsweise auf dem Prinzip eines im flüssigen Medium erzeugten grossen Geschwindigkeitsgradienten oder einer plötzlichen Richtungsänderung, oder insbesondere auf Aufprallwirkung oder gegenseitiger Reibung von Mahlkörpern, wie Metall-, Glas- oder Porzellankugeln, Kunststoffgranulat oder Sandkörner, die durch Rotation des Gefässes, oder noch wirksamer, durch Schwingungserzeuger oder rührartige Vorrichtungen, wie beispielsweise bei den Glasperlmühlen, in Bewegung gesetzt werden.

Die Mahlung wird nach bekannten Methoden durchgeführt, im allgemeinen durch Beschicken der geeigneten Mahlvorrichtung mit dem rohen Diketopyrrolopyrrol, dem Alkohol und der Base, sowie den geeigneten Mahlhilfsmitteln, Vermahlen des Systems bei einer Temperatur unter 50°C, bevorzugt zwischen 10 und 40°C und Isolieren des erhaltenen Diketopyrrolopyrrolpigments. Die Mahlung wird vorzugsweise in einer Kugelmühle durchgeführt, durch Verwendung von Sand-, Glas- oder Keramikkugeln mit einem bevorzugten durchschnittlichen Durchmesser von 0,8 bis 2,5 mm als Mahlhilfsmittel. Bevorzugt werden Mahlperlen, die aus einer kristallinen Zirkonium- und einer amorphen Kieselsäurephase der entsprechenden Oxide hergestellt werden (Produkt der Firma Quartz Products Corporation).

Wenn erwünscht können für den Alkohol/Base-Mahlvorgang Dispergiermittel, Verdünnungsmittel oder wachstumshemmende Mittel zugesetzt werden, unter der Bedingung, dass diese Additive durch das basische Medium nicht desaktiviert werden. So kann beispielsweise durch Zusatz einer geringen Menge, z.B. 3-6 Gew.-%, bezogen auf das Pigment, eines Dispergiermittels (z.B. ®DISPERBYK 160, BYK Chemie), nach dem Mahlen, ein Produkt erhalten werden, das beim Ausziehen in einer lithographischen Lackfarbe einen verbesserten Glanz im Vergleich zu einem Produkt ohne Dispergiermittel zeigt.

Das Pigment wird am besten dadurch isoliert, dass man die Pigmentaufschlämmung von den Mahlhilfsmitteln durch Filtrieren und Auswaschen des Presskuchens mit Alkohol oder durch Verdünmnung und Auswaschen mit Alkohol und/oder Wasser befreit und anschliessend den Alkohol abdestilliert. Der Alkohol kann somit zurückgewonnen werden und das Pigment durch Filtrieren der zurückgebliebenen nichtbrennbaren Aufschlämmung und Neutralwaschen mit Wasser isoliert werden.

Es kann auch von Vorteil sein bestimmte Mengen eines Texturschutzmittels vorzugsweise zum isolierten Pigment nach der Mahlung zuzugeben. Geeignete Texturschutzmittel sind beispielsweise Fettsäuren mit mindestens 18 C-Atomen, wie Stearinsäure oder Behensaure oder deren Amide oder Metallsalze, bevorzugt Magnesiumsalze, ferner Weichmacher, Wachse, Harzsäuren, wie Abietinsäure, Kolophoniumseife, Alkylphenole oder aliphatische Alkohole, wie Stearylalkohol oder vicinale Diole, wie Dodecan-1,2-diol, ferner modifizierte Kolophonium/Maleat-Harze oder Fumarsäure/Kolophonium-Harze. Texturschutzmittel kann man zweckmässig in Mengen von 0,1 bis 30 Gew.%, bevorzugt 2 bis 15 Gew.-%, bezogen auf das Endprodukt, zugegeben.

Eine charakteristische Eigenschaft der erfindungsgemäss hergestellten Pigmente ist ihre hohe Kristallinität.

Die erfindungsgemäss erhältlichen Pigmente lassen sich beispielsweise in Form von Pulver, Teig, Flushpaste und Zubereitung anwenden und eignen sich z.B. für Druckfarben, Leimfarben, Binderfarben oder Lacke aller Art, wie physikalisch und oxydativ trocknende Lacke, saure-, amin- und peroxidhärtende Lacke oder Polyurethanlacke. Die Pigmente können auch zum Färben von synthetischen, halbsynthetischen oder natürlichen makromolekularen Stoffen verwendet werden, wie Polyvinylchlorid, Polystyrol, Polyethylen, Polyestern, Phenoplasten, Aminoplasten und Gummi. Weitere Anwendungsgebiete sind das Färben von natürlichen, regenerierten oder künstlichen Fasern, wie Glas-, Silikat-, Asbest-, Holz-, Cellulose-, Acetylcellulose-, Polyacrylnitril-, Polyester-, Polyurethan- und Polyvinylchloridfasern oder deren Gemischen, eventuell zusammen mit anderen organischen oder anorganischen Pigmenten. Man erhält mit den erfindungsgemäss erhaltenen Pigmenten Drucke, Lackierungen, Anstriche, Beläge, Beschichtungen, geformte Gebilde, wie Folien, Fäden, Platten, Blöcke, Granulate und Stäbe, mit brillanter roter Farbe von hervorragender Dauerhaftigkeit.

Die erfindungsgemäss erhältlichen, roten deckenden Pigmente können zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz- oder Dispergiermittels oder durch Einrühren oder Einkneten des Pigments in ein Dispergiermittel in Gegenwart von Wasser und gegebenenfalls von organischen Lösungsmitteln oder Oelen erhalten werden. Diese Teige können beispielsweise wiederum zur Herstellung von Flushpasten, Druckfarben, Leimfarben, Kunststoffdispersionen und Spinnlösungen verwendet werden. Das Pigment kann aber auch durch Einrühren, Einwalzen, Einkneten oder Einmahlen in Wasser, organische Lösungsmittel, nicht trocknende Oele, trocknende Oele, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Die erfindungsgemäss erhältlichen Pigmente zeichnen sich nicht nur durch die ausgezeichnete Deckkraft, hervorragende Farbstärke und Reinheit des Farbtons mit sehr hoher Sättigung, sondern auch durch gute Allgemeinechtheiten, wie Licht- und Wetterbeständigkeit, Ueberlackierechtheit, Migrations- und Hitzebeständigkeit, sowie durch ausgezeichnete rheologische Eigenschaften aus.

Die nach dem erfindungsgemässen Nassmahlverfahren erhältlichen Pigmente sind vorzugsweise für die Färbung von wässrigen und/oder Lösungsmittel enthaltenden Lacken, insbesondere Automobillacken, geeignet.

Die folgenden Beispiele erläutern die Erfindung.

Beispiel 1: In einer 500 ml fassenden Breithals-Glasflasche mit Schraubendeckelverschluss werden 14 g rohes 3,6-Diphenyl-1,4-diketopyrrolo[3,4-c]-pyrrol, 15 g 30%ige wässerige Natronlauge und 180 ml Methanol zusammen mit 350 ml Keramikkugeln mit einem Durchmesser von 1,6 bis 2,5 mm und einer Zusammensetzung von 69 % ZrO₂ und 31 % SiO₂, während 48 Stunden bei 20-25°C, auf einer Rollbank so gedreht, dass die Zentrifugalkräfte grösser als die Gravitationskräfte sind, damit die Kugeln während des Drehens an der Wand der Glasflasche bleiben (70-105 U/Min.).

Danach werden die Keramikkugeln abgetrennt, die Pigmentsuspension abfiltriert, der Filterkuchen mit Methanol alkalifrei gewaschen und im Vakuumtrockenschrank bei 80°C getrocknet und gepulvert. Man erhält 13,6 g rotes Pigment, welches in Kunststoffe und Lacke eingearbeitet, deckende Ausfärbungen hoher Reinheit und Sättigung sowie ausgezeichneter Licht-, Hitze- und Wetterbeständigkeit ergibt.

Beispiel 2: Verfährt man analog wie in Beispiel 1 beschrieben, schüttelt jedoch die Glasflasche auf einer Labor-Turbula-Maschine der Firma Willy A. Bachofen, Basel während 48 Stunden, so erhält man ein Pigment mit analog guten Eigenschaften.

Beispiel 3: Verfährt man analog wie in Beispiel 2 beschrieben, verwendet jedoch anstelle von 350 ml Keramikkugeln 350 ml Glaskugeln mit einem durschschnittlichen Durchmesser von 1 mm, so erhält man ein Pigment mit analog guten Eigenschaften.

Beispiel 4: Verfährt man analog wie in Beispiel 1 beschrieben, verwendet aber anstelle von 3,6-Diphenyl-1,4-diketopyrrolo-[3,4-c]-pyrrol rohes 3,6-Di(4-chlorphenyl)-1,4-diketopyrrolo-[3,4-c]-pyrrol, so erhält man ein blaustichigrotes, deckendes Pigment, welches Ausfärbungen hoher Farbstärke und Reinheit ergibt.

Beispiel 5: Verfährt man analog wie in Beispiel 4 beschrieben, verwendet aber anstelle von 15 g 30%ige Natronlauge 7,0 g 50%ige wässerige Kalilauge, so erhält man ein rotes Pigment mit ebenso guten Eigenschaften.

Beispiel 6: In einer 100 ml fassenden Breithals-Glasflasche mit Schraubendeckelverschluss werden 2,5 g rohes 3,6-Di(4-methylphenyl)-1,4-diketopyrrolo-[3,4-c]-pyrrol, 2,5 g 30%ige wässerige Natronlauge und 37 ml Methanol zusammen mit 68 ml Keramikkugeln mit einem Durchmesser von 1,6-2,5 mm und einer Zusammensetzung von 69, % ZrO₂ und 31 % SiO₂, während 60 Stunden bei 20-25°C auf einer Rollbank so gedreht, dass die Zentrifugalkräfte grösser als die Gravitationskräfte sind, damit die Kugeln während des Drehens an der Wand der Glasflasche bleiben (70-105 U/Min).

Danach werden die Keramikkugeln abgetrennt, die Pigmentsuspension abfiltriert und der Filterkuchen mit Methanol alkalifrei gewaschen und im Vakuumtrockenschrank bei 80°C getrocknet und gepulvert. Man erhält 2,4 g rotes Pigment, welches in Kunststoffe und Lacke eingearbeitet deckende scharlachrote Ausfärbungen hoher Deckkraft, Reinheit und Sättigung sowie ausgezeichneter Licht-, Hitze- und Wetterbeständigkeiten ergibt.

Beispiel 7: Verfährt man analog wie in Beispiel 1 beschrieben, verwendet aber anstelle von Methanol wasserfreien Ethanol so erhält man ein rotes Pigment mit analog guten Eigenschaften.

Beispiel 8: In einer 2,85 l-Mühle werden 2500 g Keramikkugeln mit einem Durchmesser von 1,6-2,5 mm und bestehend aus ca. 69 % ZrO₂ und 31 % SiO₂, 50 g rohes 3,6-Diphenyl-1,4-diketopyrrolo-[3,4-c]-pyrrol, 1000 ml Methanol und 75 g 44%ige wässerige Kalilauge während 72 Stunden, einer Temperatur von 20-27°C und einer Geschwindigkeit von 65-70 U/Min. gedreht.

Die Keramikkugeln werden von der Pigmentsuspension getrennt und mit 800 ml Methanol gewaschen. Die Pigmentsuspension wird in ein Glasbehälter, ausgerüstet mit Rührer, Thermometer und Kühler, umgegossen. Wasserdampf wird in die Suspension geleitet und Methanol zusammen mit etwas Wasser abdestilliert bis die Temperatur auf 93°C steigt. Man lässt 10 Minuten rühren und filtriert ab. Der Filterkuchen wird mit Wasser neutral gewaschen und im Vakuumtrockenschrank bei 80°C getrocknet. Man erhält 48,2 g Pigment, welches gepulvert in Lacke und Kunststoffe eingearbeitet deckende rote Ausfärbungen hoher Sättigung und Reinheit ergibt.

Beispiel 9: In einer 500 ml fassenden Breithals-Glasflasche mit Schraubendeckelverschluss werden 2,5 g rohes 3,6-Diphenyl-1,4-diketopyrrolo-[3,4-c]-pyrrol, 13 ml ®TRITON B (Benzyltrimethylammoniumhydroxid 35 % in Methanol) und 30 ml Methanol zusammen mit 68 ml Keramikkugeln mit einem Durchmesser von 1,6 bis 2,5 mm und einer Zusammensetzung von 69 % ZrO₂ und 31 % SiO₂, während 48 Stunden bei 20-25°C, auf einer Rollbank so gedreht, dass die Zentrifugalkräfte grösser als die Gravitationskräfte sind, damit die Kugeln während des Drehens an der Wand der Glasflasche bleiben (70-105 U/Min.).

Danach werden die Keramikkugeln abgetrennt, die Pigmentsuspension abfiltriert, der Filterkuchen mit Methanol alkalifrei gewaschen und im Vakuumtrockenschrank bei 80°C getrocknet und gepulvert. Man erhält 2,4 g rotes Pigment, welches in Kunststoffe und Lacke eingearbeitet deckende rote Ausfärbungen hoher Reinheit und Sättigung, sowie ausgezeichneter Licht-, Hitze- und Wetterbeständigkeit ergibt.

Beispiel 10: Verfährt man analog wie in Beispiel 7 beschrieben, verwendet aber anstelle von 3,6-Di(4-methylphenyl)-1,4-diketopyrrolo-[3,4-c]-pyrrol rohes 3,6-Diphenyl-1,4-diketopyrrolo-[3,4-c]-pyrrol und 40 ml Propylenglykol anstelle von 37 ml Methanol, so erhält man ein Pigment mit analog guten Eigenschaften.

Beispiel 11: Verfährt man analog wie in Beispiel 6, verwendet aber 3,6-Di(4-tert.-butylphenyl)-1,4-diketopyrrolo-[3,4-c]-pyrrol anstelle von 3,6-Di(4-methylphenyl)-1,4-diketopyrrolo-[3,4-c]-pyrrol, so erhält man ein deckendes Pigment, das orange Ausfärbungen hoher Farbstärke und Reinheit mit ausgezeichneter Licht-, Wetter- und Hitzebeständigkeit ergibt.

Beispiel 12: Eine Mischung von
130,0 g Steatitkugeln (⌀ = 8 mm)
47,5 g eines thermohärtenden Acryllacks, bestehend aus
41,3 g Acrylharz ®VIACRYL VC 373, 60 % (VIANOVA Kunstharz AG),
16,3 g Melaminharz ®MAPRENAL TTX, 55 % (HOECHST AG),
32,8 g Xylol,
4,6 g Ethylglykolacetat,
2,0 g Butylacetat und
1,0 g ®Silikonöl A, 1 % in Xylol (BAYER AG), und
2,5 g des nach Beispiel 1 erhaltenen Pyrrolo-[3,4-c]-pyrrolpigments
wird in einer 200 ml Glasflasche mit "Twist-Off"-Verschluss während 72 Stunden auf dem Rollgestell dispergiert. Nach Abtrennen der Steatitkugeln werden
8,0 g der so dispergierten Volltonmischung,
0,6 g Aluminiumpaste ®ALCOA (60-65 % Al-Gehalt, Aluminium Corp. of America)
1,0 g Methylethylketon und
18,4 g des obigen thermohärtenden Acryllacks
gut vermischt, auf Aluminiumbleche gespritzt und anschliessend während 30 Minuten bei 130°C eingebrannt.

Man erhält sehr farbstarke deckende rote Metalleffektlackierungen mit ausgezeichneten Beständigkeiten.

Verwendet man anstelle des Pigmentes gemäss Beispiel 1 ein Pigment gemäss den Beispielen 2 bis 12, so erhält man ebenso hochwertige Ausfärbungen.

Beispiel 13: Eine Mischung von 130 g Steatitkugeln von 8 mm Durchmesser, 47,5 g Alkydmelamineinbrennlack, bestehend aus 60 g kurzöligem Alkydharz ®BECKOSOL 27-320 (Reichhold Chemie AG) 60%-ig in Xylol, 36 g Melaminharz ®SUPER-BECKAMIN 13-501 (Reichhold Chemie AG) 50%ig in Xylol:Butanol (2:1-Gemisch), 2 g Xylol und 2 g Ethylenglykolmonomethylether, und 2,5 g des nach Beispiel 1 erhaltenen Diketopyrrolo-[3,4-c]pyrrolpigments werden in einer 200 ml fassenden Glasflasche mit "Twist-Off"-Verschluss während 120 Stunden auf einem Rollgestell dispergiert. Nach Abtrennen der Steatitkugeln wird die so dispergierte Volltonmischung auf Aluminiumbleche gespritzt und anschliessend während 30 Minuten bei 130°C eingebrannt. Man erhält sehr farbstarke rote Ausfärbungen hoher Sättigung mit ausgezeichneten Beständigkeiten.

Beispiel 14: Eine Mischung bestehend aus 1,0 g des nach Beispiel 4 erhaltenen Diketopyrrolo-[3,4-c]-pyrrolpigments, 1,0 g Antioxydans (®IRGANOX 1010, CIBA-GEIGY AG) und 1000 g Polyethylen-HD Granulat (®VESTOLEN A 60-16, HUELS) wird während 15 Minuten in einer Glasflasche auf einer Rollbank vorgemischt. Danach wird die Mischung in zwei Passagen auf einem Einwellenextruder extrudiert. Das so erhaltene Granulat wird auf der Spritzgussmaschine (Allround Aarburg 200) bei 220°C zu Platten verspritzt und 5 Minuten bei 180 °C nachgepresst. Die Pressplatten weisen farbstarke rote Nuancen mit ausgezeichneten Beständigkeiten auf.

Beispiel 15: Je 0,6 g der gemäss Beispiele 1 bis 11 erhaltenen Pyrrolo-[3,4-c]-pyrrolpigmente werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugten roten PVC-Folien sind sehr farbstark, migrations- und lichtbeständig.

Beispiel 16: 1000 g Polypropylengranulat (®DAPLEN PT-55, Chemie LINZ) und 20 g eines 50%-igen Pigmentpräparates, bestehend aus 10 g des nach Beispiel 4 erhaltenen Pyrrolo-[3,4-c]-pyrrolpigments und 10 g Mg-Behenat, werden in einer Mischtrommel intensiv vermischt. Das so behandelte Granulat wird bei 260 bis 285°C nach dem Schmelzspinnverfahren versponnen. Man erhält rotgefärbte Fasern mit sehr guten Licht- und textilen Echtheiten.

Beispiel 17: In eine Lösung bestehend aus 21,5 g Celluloseacetobutyrat (25%-ig in Butylacetat), 1 g Zirkoniumoctoat 6 (®NUODEX), 12 g aromatisches Lösungsmittel SOLVESSO 150 (ESSO), 17,5 g Butylacetat, 13 g Xylol werden 8 g des nach Beispiel 4 erhaltenen Diketopyrrolo-[3,4-c]-pyrrolpigments auf einem Dispergierapparat (®DISPERMAT, Hediger, Bassersdorf) mittels Glaskugeln (⌀ 1 mm) gut dispergiert.

Nach Entfernen der Glaskugeln wird die Dispersion mit 24 g Polyesterharz ®DYNAPOL H 700 (60%-ig, DYNAMIT NOBEL) und 3 g Melaminharz ®MAPRENAL MF 650 (55%-ig, HOECHST AG) vermischt.

Der auf diese Art erhaltene Pigmentlack wird auf Aluminiumbleche gespritzt und anschliessend bei 130°C eingebrannt. Man erhält farbstarke rote Ausfärbungen mit ausgezeichnetem Glanzverhalten.

Beispiel 18: Verfährt man analog wie in Beispiel 17 beschrieben, mischt jedoch zu 12,5 g des roten Pigmentlacks noch 0,15 g DISPERBYK® 160 (BYK Chemie) zu, so erhält man rote Ausfärbungen mit noch besseren rheologischen Eigenschaften und ausgezeichnetem Glanzverhalten.

## Patentansprüche

1. Verfahren zur Herstellung von deckenden 1,4-Diketopyrrolo-[3,4-c]-pyrrole der Formel 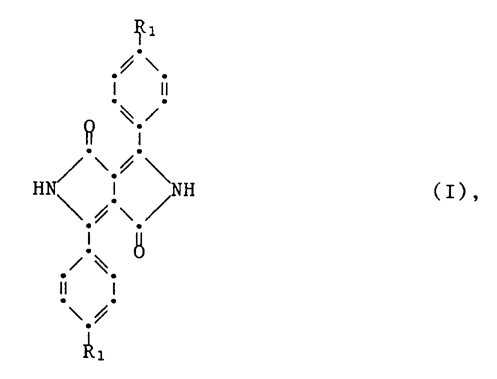 worin R₁ Wasserstoff, Chlor, Methyl oder tert.-Butyl ist, dadurch gekennzeichnet, dass das Pigment in einem Alkohol aus der Gruppe Methanol, Ethanol, Butanol, Pentanol, Ethylenglykol und Propylenglykol in Gegenwart einer Base aus der Gruppe der Alkalimetallhydroxide und quaternären Ammoniumhydroxide, bei einer Temperatur unter 50°C gemahlen wird.

2. Verfahren gemäss Anspruch 1, dadurch gekennzeichnet, dass der Alkohol Methanol oder Ethanol ist.

3. Verfahren gemäss Anspruch 1, dadurch gekennzeichnet, dass die Base Natrium- oder Kaliumhydroxid ist.

4. Verfahren gemäss Anspruch 1, dadurch gekennzeichnet, dass der Alkohol Methanol und die Base Natriumhydroxid sind.

5. Verfahren gemäss Anspruch 1, dadurch gekennzeichnet, dass der Alkohol in einer 5- bis 25-fachen Menge, bezogen auf das Gewicht des Pigments, und die Base in einer Konzentration von 2 bis 50 %, bezogen auf das Gewicht des Pigments, vorhanden sind.

6. Verfahren gemäss Anspruch 1, dadurch gekennzeichnet, dass die Mahlung bei einer Temperatur zwischen 10 und 40°C durchgeführt wird.

7. Verfahren gemäss Anspruch 1, dadurch gekennzeichnet, dass in Formel I R₁ Wasserstoff bedeutet.

## Claims

1. A process for the preparation of opaque 1,4-diketopyrrolo[3,4-c]-pyrroles of the formula 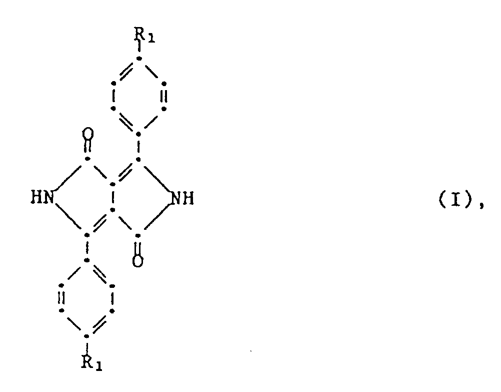 wherein R₁ is hydrogen, chlorine, methyl or tert-butyl, which comprises milling the pigment in an alcohol selected from the group consisting of methanol, ethanol, butanol, pentanol, ethylene glycol and propylene glycol and in the presence of a base selected from the group consisting of alkali metal hydroxides and quaternary ammonium hydroxides at a temperature below 50°C.

2. A process according to claim 1, wherein the alcohol is methanol or ethanol.

3. A process according to claim 1, wherein the base is sodium or potassium hydroxide.

4. A process according to claim 1, wherein the alcohol is methanol and the base is sodium hydroxide.

5. A process according to claim 1, wherein the alcohol is present in an amount of from 5 to 25 times the weight of the pigment, and the base is present in a concentration of from 2 to 50% based on the weight of the pigment.

6. A process according to claim 1, wherein milling is carried out at a temperature between 10 and 40°C.

7. A process according to claim 1, wherein in formula I R₁ is hydrogen.

## Revendications

1. Procédé pour préparer de 1,4-dicétopyrrolo-[3,4-c]-pyrroles couvrants de formule 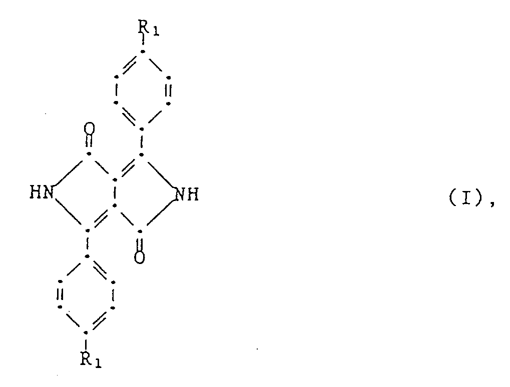 dans laquelle R₁ représente l'hydrogène, le chlore, le méthyle ou le tert-butyle, caractérisé en ce que l'on broie le pigment dans un alcool choisi dans le groupe du méthanol, de l'éthanol, du butanol, du pentanol, de l'éthylèneglycol et du propylèneglycol, en présence d'une base choisie dans le groupe des hydroxydes de métaux alcalins et des hydroxydes d'ammonium quaternaire, à une température inférieure à 50°C.

2. Procédé selon la revendication 1, caractérisé en ce que l'alcool est le méthanol ou l'éthanol.

3. Procédé selon la revendication 1, caractérisé en ce que la base est l'hydroxyde de sodium ou de potassium.

4. Procédé selon la revendication 1, caractérisé en ce que l'alcool est le méthanol et la base est l'hydroxyde de sodium.

5. Procédé selon la revendication 1, caractérisé en ce que l'alcool se trouve dans une quantité de 5 à 25 fois par rapport au poids du pigment et la base se trouve dans une concentration de 2 à 50 % calculée sur le poids du pigment.

6. Procédé selon la revendication 1, caractérisé en ce que le broyage est effectué à une température entre 10 et 40°C.

7. Procédé selon la revendication 1, caractérisé en ce que, dans la formule I, R₁ représente l'hydrogène.
